# EUROPÄISCHE PATENTSCHRIFT

(11) **EP 0 636 375 B1**
(45) Veröffentlichungstag und Bekanntmachung des Hinweises auf die Patenterteilung: **06.12.2000**
(21) Anmeldenummer: 94111439.9
(22) Anmeldetag: 22.07.1994
(51) Int. Cl.: A61L 2/16, A01N 25/34, A61M 1/00, A61B 17/00, A61C 3/00, A61D 1/00, A47G 21/00, A01N 59/16, A01N 59/20, E05B 1/00

(54) **Ausrüstungs- und Sanitärartikel für Krankenhäuser**
Equipment and sanitary devices for hospitals
Equipment et articles sanitaires pour hôpitaux

(30) Priorität: 26.07.1993 DE 4324994
(43) Veröffentlichungstag der Anmeldung: 01.02.1995
(73) Patentinhaber: Degussa-Hüls Aktiengesellschaft, 60287 Frankfurt am Main (DE)
(72) Erfinder: Breitstadt, Dr. Rolf, D-65812 Bad Soden (DE); Kaiser, Hasso, D-73529 Schwäbisch Gmünd (DE)

(56) Entgegenhaltungen:
- WO-A-93/23092
- DE-A- 4 115 390
- PATENT ABSTRACTS OF JAPAN, unexamined applications, C Sektion, Band 17, Nr. 205, 26. April 1993 THE PATENT OFFICE JAPANESE GOVERNMENT Seite 94 C1052; & JP-A-04 352 964 (MATSUSHITA ELECTRIC IND. CO. LTD.)

## Beschreibung

Die Erfindung betrifft Ausrüstungs- und Sanitärartikel, deren Oberfläche aus organischen Materialien oder nichtbioziden Metallen besteht und die beim Gebrauch mit der menschlichen Haut in Berührung kommen, zur Verwendung in Gebäuden und Anlagen, in denen die Gefahr zur Übertragung von Infektionskrankheiten besteht, wie Krankenhäuser, Altenheime, Hotels, Bäder oder öffentliche Toiletten.

Allein in deutschen Krankenhäusern sterben jährlich nach Schätzung des Bundesgesundheitsamtes bis zu 40.000 Patienten direkt oder indirekt an dort übertragenen Infektionen (Hospitalismus). Das Keimreservoir im Krankenhaus stellen beispielsweise Abflüsse, Klimaanlagen, Blumenvasen sowie Verbände dar. Der vorwiegende Übertragungsweg von Patient zu Patient geschieht über die kontaminierte Hände des Pflegepersonals. Dieser bekannten Situation tragen Ausstattungsdetails in besonders gefährlichen Bereichen (Intensivstation, OP), wie automatisch öffnende Türen oder lichtschrankengesteuerte Wasserhähne Rechnung.

Im zivilen Bereich sind insbesondere patogene Darmkeime, die orofäkal übertragen werden, wichtig. Dazu zählen u.a. Salmonellen, Hepatitis, Staphylokokken etc.

Ein wesentlicher Übertragungsmodus für diese Krankheitserreger geht ebenso über kontaminierte Hände, die mit Beschlagteilen (Türklinken, Wasserhähne) in Berührung kommen. Der Hauptübertragungsmodus auch gefährlicher Keime geschieht in Krankenhäuser über die Hände des Pflegepersonals. Generell gilt, daß die Wahrscheinlichkeit der Übertragung von Krankheitskeimen mit der Häufigkeit der Hautkontakte bzw. mit unzureichender Hygiene zunimmt.

Die Weitergabe der Infektionserreger kann auch über Sanitärartikel und Ausrüstungsgegenstände erfolgen, wie Türklinken, Wasserhähne, Armaturen oder Möbelgriffe, falls die Infektionserreger auf diesen Artikeln günstige Überlebensbedingungen vorfinden. Dies ist besonders der Fall bei Artikeln aus organischen Materialien bzw. Oberflächen (Kunststoffe, Holz, Lackschichten), aber auch bei bestimmten Metallen, wie Aluminium oder Edelstahl.

Mit flüssigen oder gasförmigen Bioziden lassen sich die Infektionserreger an ihren Ursprungsorten bekämpfen, doch sind diese Biozide einerseits flüchtig, andererseits fördern sie die Korrosion der Anlagen aufgrund ihrer Aggressivität. Zudem ist ihre wirksame Anwendung oft nicht praktikabel.

Es war daher Aufgabe der vorliegenden Erfindung, Ausrüstungs- und Sanitärartikel, deren Oberfläche aus organischen Materialien oder nichtbioziden Metallen besteht und die beim Gebrauch in Gebäuden und Anlagen, in denen die Gefahr zur Übertragung von Infektionskrankheiten besteht, mit der menschlichen Haut in Berührung kommen, so zu verändern, daß eine Übertragung von Infektionserregern möglichst zuverlässig unterbunden wird.

Diese Aufgabe wird erfindungsgemäß dadurch gelöst, daß die Oberfläche dieser Artikel mit einer bioziden KupferZinnlegierung mit etwa 50 Gew. % Zinngehalt beschichtet ist.

Die Schichtdicke dieser bioziden Metalle kann zwischen 0,01 µm bis zu mehreren Millimetern betragen. Vorzugsweise liegt sie zwischen 1 und 100 µm.

Unter Bioziden versteht man Chemikalien, die zur Bekämpfung von pflanzlichen und tierischen Schädlichen eingesetzt werden. Sie wirken vor allem auf Mikroorganismen toxisch. Auch unter den Metallen gibt es solche, deren Ionen Mikroorganismen abzutöten vermögen.

Das Aufbringen dieser Metalle auf die zu beschichtenden Artikel erfolgt mit dem bekannten Methoden, wie elektrochemische, chemische oder physikalische.

Die verstärkte Keimreduzierung konnte in einem mikrobiologischen Versuch eindrucksvoll untermauert werden. Hierbei zeigte sich eine dramatische Keimzahlreduktion schon nach einer Stunde auf Substraten, die mit einer Kupfer/Zinn-Legierung beschichtet waren im Vergleich zu Edelstahl, eloxiertem Aluminium, pulverlackiertem Aluminium oder Messing vernickelt. Erstaunlicherweise war die Keimzahlreduzierung auf der Kupfer-Zinn-Legierung auch wesentlich höher als die auf Silber. Dieser Versuch wurde mit zwei unterschiedlichen Keimzahlansätzen sowie repräsentativen und standardisierten Keimstämmen durchgeführt. Die Absterberaten wurden jeweils nach 1, 6 und 24 Stunden ermittelt.

Kupfer/Zinn-Oberflächen haben darüberhinaus den Vorteil, daß die Anlaufbeständigkeit der Substrate wesentlich günstiger ist als beispielsweise von Kupfer oder Silber (Handschweiß).

## Patentansprüche

1. Ausrüstungs- und Sanitärartikel, deren Oberfläche aus organischen Materialien oder nichtbioziden Metallen besteht und die beim Gebrauch mit der menschlichen Haut in Berührung kommen, zur Verwendung in Gebäuden und Anlagen, in denen die Gefahr zur Übertragung von Infektionskrankheiten besteht,
**dadurch gekennzeichnet**,
daß die Oberfläche dieser Artikel mit einer Kupfer-Zinn-Legierung mit etwa 50 Gew. % Zinngehalt beschichtet ist.

2. Ausrüstungs- und Sanitärartikel nach Anspruch 1,
**dadurch gekennzeichnet**,
daß die Schichtdicke zwischen 1 und 100 µm beträgt.

## Claims

1. Equipment and sanitary articles, the surfaces of which consist of organic materials or non-biocidal metals and which when in use come into contact with the human skin, for use in buildings and plants wherein there is the risk of transmission of infectious diseases,
characterised in that
the surfaces of these articles are coated with a copper-tin alloy having a tin content of about 50 wt.%.

2. Equipment and sanitary articles according to claim 1,
characterised in that
the layer thickness is between 1 and 100 µm.

## Revendications

1. Equipement et articles sanitaires dont la surface est réalisée en matières organiques ou en métaux non biocides et qui à l'utilisation arrivent en contact avec la peau humaine, destinés à être utilisés dans des immeubles et des installations dans lesquels existe le risque de transmission de maladies infectieuses,
caractérisés en ce qu'
on revêt la surface de ces articles avec un alliage cuivre-zinc ayant une teneur en zinc d'environ 50 % en poids.

2. Epuisement et articles selon la revendication 1,
caractérisés en ce que
l'épaisseur de la couche est comprise entre 1 µm et 100 µm.
